# FASCICULE DE BREVET EUROPEEN

(11) **EP 3 806 819 B1**
(45) Date de publication et mention de la délivrance du brevet: **13.07.2022**
(21) Numéro de dépôt: 19731940.3
(22) Date de dépôt: 14.06.2019
(51) Int. Cl.: A61K 8/9722, A61Q 19/00, A61Q 19/08, C12N 1/12, A61K 8/20, A61K 8/60

(54) **PROCEDE D'OBTENTION D'UN EXTRAIT AQUEUX DE DUNALIELLA SALINA ET SES UTILISATIONS COSMETIQUES**
VERFAHREN ZUR GEWINNUNG EINES WÄSSRIGEN EXTRAKTES AUS DUNALIELLA SALINA UND KOSMETISCHE VERWENDUNGEN DAVON
METHOD FOR OBTAINING AN AQUEOUS EXTRACT OF DUNALIELLA SALINA AND COSMETIC USES OF SAME

(30) Priorité: 15.06.2018 FR 1870710
(43) Date de publication de la demande: 21.04.2021
(73) Titulaire: ISP Investments LLC, Wilmington, Delaware 19805 (US); Seacret Spa Ltd., 6209717 Tel Aviv (IL)
(72) Inventeur: IMBERT, Isabelle, 06400 CANNES (FR); LE MESTR, Audrey, 06600 ANTIBES (FR); CHABERT, Rachel, 06130 GRASSE (FR); AFRIAT STALOFF, Isabelle, 9841057 MAALE ADUMIM (IL); YADIN, Boaz, 6209717 TEL AVIV (IL); OGER, Elodie, 06600 Antibes (FR)
(74) Mandataire: Axe PI
(86) Numéro de dépôt international: PCT/EP2019/065655
(87) Numéro de publication internationale: WO 2019/238914

(56) Documents cités:
- WO-A2-2008/104570
- FR-A1- 2 657 012
- US-B1- 6 248 340
- GLADUE ET AL.: "Microalgal feeds for aquaculture", JOURNAL OF APPLIED PHYCOL, vol. 6, 1 avril 1994 (1994-04-01), pages 131-14, XP008175354, cité dans la demande
- OREN A ET AL: "Factors determining the development of algal and bacterial blooms in the Dead Sea: a study of simulation experiments in outdoor ponds", FEMS MICROBIOLOGY LETTERS, WILEY-BLACKWELL PUBLISHING LTD, GB, vol. 31, no. 4, 1 août 1985 (1985-08-01), pages 229-237, XP023920426, ISSN: 0378-1097, DOI: 10.1111/J.1574-6968.1985.TB01154.X [extrait le 1985-08-01]

## Description

### Domaine technique

La présente invention concerne le domaine de la cosmétique. Elle concerne plus particulièrement un procédé d'obtention d'un extrait aqueux d'une microalgue *Dunaliella salina,* les extraits obtenus par un tel procédé et les compositions cosmétiques comprenant de tels extraits, ainsi que leurs utilisations cosmétiques pour le soin de la peau, du cuir chevelu et des phanères.

La peau est un organe vital composé de plusieurs couches (derme, couches prolifératives et *stratum corneum*), qui recouvrent toute la surface du corps et assure des fonctions protectrices, sensibles, immunitaires, métaboliques ou thermorégulatrices. La peau constitue l'interface entre l'organisme et l'environnement extérieur. Elle a pour but de protéger l'organisme des agressions extérieures mais également de lutter contre la déshydratation en limitant la diffusion de l'eau. Cette barrière cutanée est en grande partie assurée par l'épiderme. La peau, comme les autres organes, est sujette au vieillissement.

L'aspect de la peau peut être modifié par des altérations internes (vieillissement intrinsèque, maladies et changements hormonaux tels que la grossesse) ou externes (facteurs environnementaux, tels que la pollution, la lumière du soleil, les pathogènes, les variations de température, etc.). Suite à ces altérations, peuvent apparaître des rides et des ridules, des défauts de pigmentation, une sécheresse ou même une déshydratation de la peau, un amincissement de l'épiderme, une élastose, des imperfections, des taches de vieillesse, etc. Tous ces changements affectent non seulement la peau, mais aussi les annexes kératiniques tels que les ongles et les cheveux.

Par ailleurs, le terme microalgue (ou microphyte), désigne les algues microscopiques. *Dunaliella salina* est un type de microalgue verte, unicellulaire, bi-flagellée et halophile appartenant à la famille des Chlorophycée qui comprend environ 30 genres dont celui de *Dunaliella.* Il existe environ une dizaine d'espèces du genre *Dunaliella,* les plus connues et étudiées étant D. *tertiolecta,* D. *media,* D. *euchlora,* D. *minuta, D. parva,* et D. *viridis.* Il faut souligner que toutes les espèces mentionnées ci-avant ne tolèrent pas des concentrations aussi élevées de sel que D. salina. Certaines espèces de *Dunaliella* sont des organismes marins qui n'ont jamais été signalés dans des environnements hypersalins.

Peu d'organismes peuvent donc survivre, comme *Dunaliella salina,* dans des conditions présentant d'aussi fortes concentrations en sel. Les espèces de *Dunaliella* sont en effet capables de tolérer des concentrations variables de NaCl allant de 0,2% à environ 35%. Aussi, dans la mer Morte, on retrouve majoritairement les espèces de *Dunaliella* et plus particulièrement *Dunaliella salina* (Volacani BE, 1944). La mer Morte se situe le long de la faille syro africaine. Elle est le résultat d'un phénomène géologique qui se constitua il y a trois millions d'années. Lors d'un gigantesque bouleversement naturel, plusieurs strates de terres riches en minéraux vinrent à la surface, des sources jaillirent donnant naissance à une vallée et un lac situé sous le niveau de la mer. L'évaporation qui s'est poursuivie pendant des millénaires a eu pour résultat de concentrer les sels et les minéraux dans les eaux du lac que l'on appelle aujourd'hui la mer Morte à un niveau rencontré dans nulle autre mer ou océan. Ainsi, les eaux de la mer Morte contiennent 3 fois plus de sodium, 30 fois plus de magnésium, 16 fois plus de potassium et 36 fois plus de calcium que la mer Méditerranée par exemple. La microalgue *Dunaliella salina* est l'une des rares espèces à pouvoir vivre dans un environnement où la salinité est si importante. *Dunaliella salina* a également une très grande tolérance au pH allant de pH 1 à pH 11 et est également capable de supporter des températures inférieures à 0°C et supérieures à 38°C.

Les *Dunaliella* ne possédant pas de paroi cellulaire rigide, elles produisent une quantité importante de glycérol pour faire face à une forte concentration en sel et fournir une protection contre la pression osmotique. Ainsi, le glycérol agit comme un « soluté compatible » qui protège en particulier les enzymes contre l'inactivation et l'inhibition (Brown et Borowitzka, 1979). De plus, pour survivre à l'exposition à une lumière parfois intense, ces microalgues synthétisent des caroténoïdes. Cette grande quantité de caroténoïdes fournit également une activité antioxydante. *Dunaliella salina* a d'abord été remarquée dans les bassins d'évaporation saline dans le sud de la France en 1838 par Michael Felix Dunal et nommée d'après son découvreur par Teodoresco en 1905. Après cette découverte, *Dunaliella salina* est ainsi devenue un organisme modèle pour l'étude de l'adaptation au sel. L'établissement de concept de solutés compatibles avec les composés organiques pour obtenir un équilibre osmotique est en effet largement basé sur l'étude des espèces de *Dunaliella.*

La forme de la cellule chez les espèces de *Dunaliella* varie d'ellipsoïde, ovoïde, cylindrique, pyriforme et fusiforme à presque sphérique. Les cellules d'une espèce donnée peuvent changer de forme selon les conditions environnementales, devenant souvent sphériques dans des conditions défavorables.

La taille des cellules peut également varier dans une certaine mesure avec les conditions de croissance et l'intensité lumineuse (Marano, 1976; Riisgård, 1981 ; Einsphar et al., 1988).

Les microalgues sont consommées depuis des milliers d'années dans le monde. Par exemple, des traces de la consommation de diverses espèces de microalgues au Mexique du temps des Aztèques ont été retrouvées. L'Europe et les pays industrialisés utilisent des microalgues en tant que compléments alimentaires pour lutter contre la malnutrition, ainsi que pour l'aquaculture. Ces microalgues, en particulier *Dunaliella* salina sont notamment utilisées pour des solutions alternatives de carburant, appelé biofuel, qui représentent l'utilisation première de cette microalgue dans le monde. Elles sont dans ces cas-là cultivées en milieu extérieur, dans des bassins de type « raceway », ou en milieu fermé, dans des photobioréacteurs. En effet, les espèces de *Dunaliella* et en particulier *Dunaliella salina,* sont connues pour leur capacité à produire et accumuler de grandes quantités de lipides, β-carotène sous forme de gouttelettes, de sorte que les cellules paraissent rouge-orangé plutôt que vertes. Ces molécules produites par les *Dunaliella* ont des applications dans des domaines aussi variés que la production de biocarburants, la cosmétique ou la nutraceutique.

### Technique antérieure

Aujourd'hui, le mode de culture le plus courant pour cultiver des microalgues et notamment *Dunaliella salina* est la culture en condition autotrophique en bassins ouverts. Dans ce type de culture, les microalgues captent l'énergie lumineuse et utilisent le CO2 comme source de carbone, en réalisant la photosynthèse. Il suffit alors que le milieu de culture contienne des éléments minéraux pour que les microalgues se multiplient. Cependant, la culture photo-autotrophique présente un faible rendement. La production de biomasse est en effet sévèrement limitée en raison de l'auto-ombrage due à l'augmentation de la biomasse au cours de la culture qui empêche la disponibilité de la lumière vers la fin de croissance. Ce type de culture requiert en outre un coût important.

Certaines microalgues comme les *Dunaliella* peuvent également pousser en condition mixotrophique. Dans ce cas, elles utilisent à la fois une source de carbone organique et l'énergie lumineuse.

Enfin, les *Dunaliella* peuvent également se développer dans des conditions de culture dites hétérotrophiques : les microalgues sont cultivées dans l'obscurité, dans des bioréacteurs fermés ou ouverts. Elles doivent alors disposer d'une source de carbone organique, comme par exemple l'éthanol, l'acétate et le glucose.

Toutes ces méthodes de culture sont largement décrites dans la littérature scientifique (Bumbak et al.,2011).

L'avantage de la production en bioréacteurs est qu'elle peut être bien maîtrisée. Il est en effet possible de contrôler tous les paramètres de culture, pH, température, milieu de culture, taux de sucre ou autres molécules nécessaires à la croissance des microalgues. L'inconvénient est que la culture en bioréacteur représente un coût pour se doter de tout le matériel permettant ce mode de culture.

De façon générale, le milieu de culture en condition hétérotrophique des microalgues telles que les *Dunaliella,* doit contenir des molécules indispensables à leur croissance en l'absence de lumière. Les milieux de culture utilisés sont souvent des milieux de culture synthétiques contenant du sel, des macro et micro-nutriments, des vitamines, des sels minéraux, tels que retrouvé dans le milieu modifié de Johnson (Johnson et al., 1968 ; Borowitzka, 1988) très largement utilisé pour la culture de *Dunaliella.* Ces milieux doivent assurer la croissance des *Dunaliella* en apportant les hydrates de carbone. Les microalgues *Dunaliella* ont aussi besoin, pour leur croissance, d'une source d'azote, qui peut être apportée soit sous forme de nitrate, d'ammoniac, d'urée ou encore par l'utilisation d'un extrait de levure fréquemment utilisé pour la culture de bactéries, de levures. Le phosphore représente aussi un nutriment majeur pour la croissance des microalgues étant donné son implication dans plusieurs processus cellulaires comme les transferts d'énergie et la synthèse d'acides nucléiques et de phospholipides. Ce phosphore est principalement assimilé sous forme inorganique (H2PO4- ou HPO42-) (Barsanti et Gualtieri, 2006).

On connait par exemple le document JP2003325165 qui décrit une méthode de culture de *Dunaliella* dans de l'eau de mer concentrée pour son utilisation en thalassothérapie. *Dunaliella* peut être utilisée sous forme de poudre ou de pâte.

On connait également la publication Gladue et al. (« Microalgal feeds for aquaculture », Journal of applied phycol, Kluwer, Dordrecht, NL, vol.6 no.2, 1 avril 1994, pages 131-14) qui divulgue un milieu de culture pour microalgue comprenant de l'eau de mer (80%), du glucose (1,8%) et de l'extrait de levure (0,05%). D. salina est cultivée dans ce milieu de culture en absence de lumière sous agitation modérée pendant une période d'au moins 24 heures, le pH étant de 7,5-8.

On connait également le document WO2013058431 qui décrit une méthode de culture de *Dunaliella* dans de l'eau de mer naturelle dans laquelle sont ajoutés du charbon et du NaOH.

On connait des documents brevets (par exemple CN102851214, CN102936569, CN104988065, KR2006000307, ou CN 1923994) décrivant différents modes de culture de *Dunaliella salina* dans des milieux comprenant notamment une source d'azote (KNO3, NH4NO, CO(NH2)2, (NH4)2CO3, NH4NO3, NaNO3), une source de phosphore et/ou de potassium (KH2PO4, KNO3, Na3PO4), une source de carbone inorganique (NaHCO3), du sel (NaCl ou de l'eau de mer) et éventuellement des vitamines ou des composés ioniques pour améliorer la croissance de *Dunaliella salina* et la production de bêta-carotène.

On connait plus particulièrement le document brevet CN 1446904 qui décrit la mise en culture de *Dunaliella salina* pendant 2 à 3 semaines à partir de poudre de *Dunaliella salina* dans de l'eau salée, à la lumière, à une température comprise entre 10 et 35°C, le milieu de culture présentant un pH du milieu compris entre 6 et 8 et comprenant du NaH2PO4 et CH4N2O. Cette algue peut être utilisée en tant que soins cosmétiques ou thérapeutiques et à des fins de génie génétique.

Gladue et al., "Microalgal feeds for aquaculture", 1994, divulgue des méthodes de culture des microalgues sous conditions hétérotrophiques.

*Dunaliella salina* est ainsi connue pour être utilisée notamment en cosmétique en partie pour l'utilisation des β-carotènes qu'elle contient, reconnus pour leur activité antioxydante et indispensables à la synthèse de la vitamine A. Le β-carotène protège la peau des rayons nocifs du soleil et se transforme en vitamine A dans la peau, ce qui permet son renouvellement. *Dunaliella salina* contient également beaucoup d'acides aminés (incluant la glycine et l'alanine) et des acides gras essentiels enrichis en vitamine E et B. Ces éléments sont fondamentaux pour lutter contre le vieillissement de la peau. *Dunaliella salina* contient aussi une quantité élevée de glycérol qui a des vertus absorbantes et de rétention de l'eau importante.

Par ailleurs, malgré les nombreux produits cosmétiques anti-âge sur le marché pour le traitement de la peau, les produits dits « non naturels » et synthétisés chimiquement peuvent être perçus comme dangereux pour l'environnement ainsi que pour les personnes. Les produits naturels sont en revanche généralement mieux perçus.

Bien que de nombreux produits naturels extraits de plantes ou d'algues soient connus pour contenir des phyto-composés qui peuvent avoir des effets bénéfiques sur la peau, il reste un besoin de nouvelles compositions cosmétiques efficaces appliquées topiquement qui présentent des effets anti-âge, hydratant pour la peau et les cheveux, utilisant des ingrédients naturels comme agent actif.

L'extrait de *Dunaliella salina* décrit dans la présente invention, a pour avantage d'être obtenu à partir de culture de *Dunaliella salina* réalisée en condition hétérotrophique, en présence d'un milieu de culture complètement naturel, contrairement à d'autres extraits déjà connus sur le marché et utilisant des milieux de culture synthétiques. L'intérêt de la culture hétérotrophique permet de réaliser la culture de *Dunaliella salina* en absence de lumière, ce qui permet un mode de culture plus facilement adaptable à grande échelle sans nécessité d'appareils spécifiquement dédiés à la culture de microorganismes tels que les microalgues.

### Problème technique

Considérant ce qui précède, un problème que se propose de résoudre la présente invention est de fournir une nouvelle méthode de culture de *Dunaliella salina* pour obtenir un extrait enrichi en composés d'intérêt pour le soin de la peau. De tels extraits naturels selon l'invention présentent ainsi des effets anti-âge et hydratant améliorés.

### Solution technique

La solution à ce problème posé a pour premier objet un procédé d'obtention d'un extrait aqueux d'une microalgue *Dunaliella salina,* dans lequel ladite microalgue est cultivée en absence de lumière dans un milieu de culture comprenant un extrait de levure, du sucre et du sel suivant les étapes selon lesquelles :
a) on solubilise un extrait de levure dans de l'eau ;
b) on ajoute du glucose dans le mélange obtenu en a) ;
c) on ajoute du sel de la mer Morte ;
d) après totale solubilisation du sel dans le mélange obtenu en b), le milieu de culture ainsi obtenu présente un pH compris entre 5 et 8 dans lequel on ajoute la microalgue *Dunaliella salina ;*
e) on maintient le mélange obtenu en d) sous agitation modérée, à l'obscurité et à température ambiante, pendant une période d'au moins 12h, pour permettre la fermentation de la microalgue *Dunaliella salina ;*
f) le mélange de *Dunaliella salina* fermenté obtenu en e) est broyé puis filtré afin de séparer les matières solubles et insolubles ;
g) on récupère un extrait brut aqueux soluble dans lequel on rajoute du sel de la mer Morte ou un conservateur tel que du sodium benzoate ;
h) on réalise une filtration stérilisante avec un seuil de porosité inférieur ou égal à 0,2 µm; et
i) on obtient un extrait aqueux de *Dunaliella salina* fermenté dans lequel le pH est compris entre 3,5 et 4,5.

Elle a pour deuxième objet un extrait aqueux de *Dunaliella salina* susceptible d'être obtenu par le procédé selon l'invention, comprenant en poids du poids total de l'extrait, de 15 à 25 g/kg de poids sec, 0,1 à 2 g/kg de fragments protéiques, 0,3 à 3 g/kg de sucres, 0,5 à 3 g/kg d'acides aminés, et 20 à 150 mg/kg de composés phénoliques.

Elle a pour troisième objet une composition comprenant, en tant qu'agent actif anti-âge, une quantité efficace d'un extrait aqueux de *Dunaliella salina* selon l'invention, et un milieu physiologiquement acceptable.

Enfin, l'invention a pour dernier objet l'utilisation cosmétique d'une composition selon l'invention pour le soin de la peau, du cuir chevelu et des phanères.

### Avantages apportés

Les inventeurs ont développé une nouvelle méthode de culture de *Dunaliella salina* basée sur le système de culture hétérotrophique, dans lequel *Dunaliella salina* produit des composés d'intérêt par fermentation.

L'un des avantages de la présente invention est que la microalgue *Dunaliella salina* est utilisée vivante, cultivée en condition hétérotrophique dans un milieu de culture spécifique et complètement naturel, composé d'un extrait de levure, de sucre et de sel de la mer Morte. Tous les éléments utilisés nécessaires à la croissance de la microalgue sont naturels, aucune molécule synthétique n'étant additionnée au milieu de culture. La fermentation va s'établir car le milieu nutritif contient tous les composés nécessaires à la croissance de la microalgue. Les différents composés vont être métabolisés par la microalgue *Dunaliella salina,* pour lui permettre de se développer. En effet, l'utilisation d'un extrait de levure permet d'apporter les sources azotées nécessaires à la croissance de la microalgue, l'ajout de glucose permet d'apporter la source carbonée également nécessaire à la croissance de la microalgue. De plus, le fait que *Dunaliella salina* doit évoluer dans un milieu salin est rendu possible par l'ajout de sel de la mer Morte permettant d'apporter tous les minéraux essentiels. L'intérêt d'utiliser le sel de la mer Morte vient également de ses propriétés bénéfiques sur la peau reconnues depuis longtemps en cosmétique. Ses eaux très riches en oligo-minéraux lui confèrent des vertus apaisantes et bienfaisantes sur la peau. En effet, la composition des eaux de la mer Morte est unique et contient des concentrations en magnésium, potassium, silice, sodium et calcium plus élevés que toutes les autres eaux salées du monde, océans inclus.

La fermentation des microalgues *Dunaliella salina* dans ces conditions de culture selon l'invention permet d'obtenir après extraction de la biomasse cellulaire, un extrait final hautement enrichi en différents composés d'intérêt issus de la microalgue mais aussi de son milieu de culture. Ce dernier peut être conservé au cours de l'extraction, car il est composé exclusivement de molécules naturelles. Ceci permet de proposer un nouvel extrait de *Dunaliella salina,* différent d'un extrait réalisé uniquement à partir de *Dunaliella salina* séchées.

L'extrait de *Dunaliella salina* fermenté selon l'invention est riche en composés d'intérêt tels que des acides aminés, des monosaccharides, des protéines et des peptides. Associés aux sels minéraux du sel de la mer Morte, l'ensemble de ces molécules hydrosolubles, connues pour leurs effets bénéfiques sur la peau, contribue à l'efficacité améliorée de l'extrait selon l'invention par rapport à un extrait de *Dunaliella salina* non fermenté.

Dans cette description, à moins qu'il n'en soit spécifié autrement, il est entendu que, lorsqu'un intervalle est donné, il inclut les bornes supérieure et inférieure dudit intervalle.

### Brève description des dessins

L'invention et les avantages qui en découlent seront mieux compris à la lecture de la description et des modes de réalisation non limitatifs qui suivent, illustrés au regard des dessins annexés dans lesquels :
La Figure 1 représente la croissance de *Dunaliella salina* en culture suivant le procédé de l'exemple 1 selon l'invention.
La Figure 2 représente la consommation en hexose de *Dunaliella salina* en culture suivant le procédé de l'exemple 1 selon l'invention.
La Figure 3 représente l'effet de la présence de *Dunaliella salina* sur la concentration en acides aminés (protéines) contenus dans un milieu de culture suivant le procédé de l'exemple 1 selon l'invention.
La Figure 4 représente l'évaluation d'un extrait aqueux de *Dunaliella salina* fermenté obtenu suivant le procédé de l'exemple 1 selon l'invention sur la fonction barrière et les mouvements de l'eau sur de la peau ex *vivo.*

### Description des modes de réalisation

L'invention concerne un procédé mis en œuvre pour l'obtention d'un extrait aqueux de *Dunaliella salina* fermenté à partir de *Dunaliella salina* vivantes, cultivées en condition de culture hétérotrophique, dans un milieu comprenant un extrait de levure, du sucre et du sel, et en l'absence de lumière. La culture s'effectue avantageusement en milieu ouvert.

Par *Dunaliella,* on entend toute les microalgues de l'espèce *Dunaliella. Dunaliella* est une microalgue capable de se développer dans différents types d'environnement : halotrophique (énergie de la lumière), hétérotrophique (énergie provenant de l'apport de carbone), mixotrophique (mélange de lumière et de carbone).

Préférentiellement, l'extrait est obtenu à partir de la fermentation de *Dunaliella salina.*

Par fermentation, on entend la mise en culture de la microalgue dans un milieu nutritif spécifique selon l'invention, contenant notamment une source de carbone organique, que la microalgue va métaboliser pour croitre.

Plus particulièrement, on entend par fermentation des microalgues *Dunaliella salina* en condition hétérotrophique, la mise en culture dans un milieu contenant tous les éléments nutritifs nécessaires à la croissance de la microalgue et où l'apport de sources carbonées, notamment du glucose, va permettre la multiplication de la microalgue dans un environnement dépourvu de lumière (Barclay et al., 1994, Heterotrophic production of long chain omega-3 fatty acids utilizing algae and algae-like microorganisms ; Garcia et al., 2013, A process for biodiesel production involving the heterotrophic fermentation of Chlorella protothecoides with glycerol as the carbon source).

L'extrait aqueux de la microalgue *Dunaliella salina* selon l'invention est obtenu à partir du procédé comprenant les étapes suivantes :
a) on solubilise un extrait de levure dans de l'eau ;
b) on ajoute du glucose dans le mélange obtenu en a) ;
c) on ajoute du sel de la mer Morte ;
d) après totale solubilisation du sel dans le mélange obtenu en b), le milieu de culture ainsi obtenu présente un pH compris entre 5 et 8 dans lequel on ajoute la microalgue *Dunaliella salina ;*
e) on maintient le mélange obtenu en d) sous agitation modérée, à l'obscurité et à température ambiante, pendant une période d'au moins 12h, pour permettre la fermentation de la microalgue *Dunaliella salina ;*
f) le mélange de *Dunaliella salina* fermenté obtenu en e) est broyé puis filtré afin de séparer les matières solubles et insolubles ;
g) on récupère un extrait brut aqueux soluble dans lequel on rajoute du sel de la mer Morte ou un conservateur tel que du sodium benzoate ;
h) on réalise une filtration stérilisante avec un seuil de porosité inférieur ou égal à 0,2 µm; et
i) on obtient un extrait aqueux de *Dunaliella salina* fermenté dans lequel le pH est compris entre 3,5 et 4,5.

*Dunaliella salina* est l'une des rares espèces vivantes capable de vivre dans la mer Morte. En effet, alors que la salinité moyenne de l'eau de mer oscille entre 2 et 4%, celle de la mer Morte est d'approximativement 27,5% (soit 275 grammes par litre).

La mer Morte est un lac d'eau salée. Son eau est très riche en minéraux et en oligo-éléments et possède de nombreuses vertus. Le sel naturel de la mer Morte possède vingt-six minéraux vitaux, parmi lesquels on trouve le calcium, le potassium et le magnésium. Le sel naturel de la mer Morte, contient plus particulièrement en moyenne : potassium (120 000 mg/l), magnésium (85 000 mg/l), chlore (38 000 mg/l), sodium (23 000 mg/l), calcium (22 000 mg/l), brome (5600 mg/l), carbonate, chrome, phosphore, fer... Le sel de la mer Morte de par sa richesse en différents minéraux essentiels, est très utilisé en cosmétique, et reconnu notamment pour ses vertus bénéfiques sur la peau. Il a une action désinfectante et réparatrice sur la peau (Proksch et al., Int J Dermatol. 2005 ; 44(2) :151-7).

Le calcium qu'il contient favorise les échanges cellulaires.

Le magnésium contribue au ralentissement du processus de vieillissement et permet de bien détendre les muscles. Il soulage également les articulations. Il est considéré comme un antistress naturel.

Le potassium permet d'équilibrer l'hydratation de la peau. Il favorise donc le rétablissement de l'équilibre en eau.

Dans une première étape a) du procédé selon l'invention, on dissout un extrait de levure, par exemple sous forme de poudre, avec de l'eau, préférentiellement dans un ratio extrait de levure / eau de 0,1 à 2% en poids/poids, plus préférentiellement dans un rapport de 0,2%.

L'eau utilisée est une eau distillée, déminéralisée ou encore une eau riche en sels minéraux et/ou oligo-éléments, préférentiellement une eau distillée.

De préférence, l'extrait de levure est un extrait de Saccharomyces cereviseae.

L'extrait de levure peut être sous forme de poudre ou d'extrait de levure fraiche. De préférence, l'extrait de levure est sous forme de poudre.

On ajoute ensuite dans une étape b) du glucose dans le mélange obtenu en a).

La concentration en glucose est préférentiellement comprise entre 0,1 et 4%, et plus préférentiellement de 0,2%. Cette concentration est choisie pour optimiser le rendement de fermentation de la microalgue *Dunaliella salina.*

Dans une étape c), une fois que le glucose et l'extrait de levure sont solubilisés, on ajoute du sel de la mer Morte, préférentiellement entre 1 et 5%, plus préférentiellement 2,6%, qui va permettre d'apporter à la microalgue tous les sels minéraux essentiels à son développement, et obtenir une salinité de l'eau indispensable pour le développement de la microalgue, le pH du mélange devant être compris entre 5 et 8 ;
Par exemple, on ajuste le pH par ajout d'une solution d'acide chlorhydrique (HCl) ou encore n'importe quel acide pouvant réguler le pH qui soit compatible avec une utilisation cosmétique tel que l'acide citrique ou lactique.

Ce mélange va constituer un milieu de culture optimal pour accueillir la microalgue, et lui fournir tous les éléments nutritifs nécessaires à son développement, sa croissance, et de fait sa division cellulaire.

Dans une étape d), on ajoute la microalgue *Dunaliella salina* dans cette solution nutritive soluble présentant un pH compris entre 5 et 8.

Selon un mode de réalisation avantageux du procédé selon l'invention, on ajoute préférentiellement entre 0,5% et 10% d'inoculum de *Dunaliella salina* au milieu de culture, plus préférentiellement entre 1% et 5%, encore plus préférentiellement 2%.

Dans une étape e), on maintient le mélange obtenu en d) sous agitation modérée, à l'obscurité et à température ambiante, pendant une période d'au moins 12h, pour permettre la fermentation de la microalgue *Dunaliella salina.*

La fermentation est réalisée lorsque *Dunaliella salina* est mise en culture dans un tel milieu nutritif spécifique selon l'invention contenant un extrait de levure, du glucose et du sel de la mer Morte, dans de l'eau à température ambiante ou avec de l'eau chauffée à une température maximale de 35°C pendant au moins 12h, avantageusement en milieu ouvert.

Dans une étape f), le mélange de *Dunaliella salina* fermenté obtenu en e) est broyé puis filtré afin de séparer les matières solubles et insolubles de manière à récupérer dans une étape g) un extrait brut aqueux soluble dans lequel on rajoute du sel de la mer Morte ou un conservateur tel que le sodium benzoate.

L'ajout de sel de la mer Morte permet de jouer le rôle de conservateur.

Dans une étape h), on réalise une filtration stérilisante avec un seuil de porosité inférieur ou égal à 0,2 µm.

Enfin dans une étape i), on obtient un extrait aqueux de *Dunaliella salina* fermenté dans lequel le pH est compris entre 3,5 et 4,5, préférentiellement entre 3,5 et 4,0, plus préférentiellement le pH est de 4,0.

Préférentiellement, dans les étapes d) et i), le pH est contrôlé et éventuellement réajusté par l'ajout d'une solution d'acide chlorhydrique (HCl) ou de soude (NaOH).

L'extrait aqueux de *Dunaliella salina* susceptible d'être obtenu par le procédé selon l'invention est composé notamment d'acides aminés, de sucres, de fragments protéiques et de composés phénoliques.

L'extrait ainsi obtenu selon l'invention est une solution claire et brillante.

Préférentiellement, un tel extrait aqueux de *Dunaliella salina* susceptible d'être obtenu par le procédé selon l'invention comprend en poids du poids total de l'extrait, de 15 à 25 g/kg de poids sec, 0,1 à 2 g/kg de fragments protéiques, 0,3 à 3 g/kg de sucres, 0,5 à 3 g/kg d'acides aminés et 20 à 150 mg/kg de composés phénoliques.

Préférentiellement, un extrait aqueux de *Dunaliella salina* susceptible d'être obtenu par le procédé selon l'invention comprend en poids du poids total de l'extrait, 0,5 - 1 g/kg de fragments protéiques, 1 - 2,5 g/kg de sucres, 0,6 - 2 g/kg d'acides aminés et 40 - 100 mg/kg de composés phénoliques.

L'extrait aqueux selon l'invention ne comprend pas de quantité détectable de molécules lipophiles telles que les caroténoïdes.

Un autre objet de l'invention concerne une composition comprenant, en tant qu'agent actif anti-âge, une quantité efficace d'un extrait aqueux de *Dunaliella salina* susceptible d'être obtenu par le procédé selon l'invention, et un milieu physiologiquement acceptable.

Par quantité efficace, on désigne la quantité minimum d'extrait selon l'invention qui est nécessaire pour obtenir l'activité de l'extrait, en particulier cosmétique et plus particulièrement pour améliorer l'aspect de la peau, lutter contre les signes du vieillissement cutané ou pour l'amélioration de l'hydratation de la peau, sans que cette quantité soit toxique.

Un milieu physiologiquement acceptable désigne un véhicule adapté pour une mise en contact avec les couches externes de la peau ou des muqueuses, sans toxicité, irritation, réponse allergique indue et similaire ou réaction d'intolérance, et proportionné à un rapport avantage/risque raisonnable.

A titre de milieu physiologiquement acceptable communément utilisé dans le domaine d'application envisagé, on peut citer par exemple des adjuvants nécessaires à la formulation, tels que des solvants, des épaississants, des diluants, des antioxydants, des colorants, des filtres solaires, des agents auto-bronzants, des pigments, des charges, des conservateurs, des parfums, des absorbeurs d'odeur, des huiles essentielles, des vitamines, des acides gras essentiels, des tensioactifs, des polymères filmogènes, etc.

Préférentiellement, la composition selon l'invention comprend l'extrait aqueux de *Dunaliella salina* susceptible d'être obtenu par le procédé selon l'invention à une concentration de 0,1 à 10% en poids par rapport au poids total de la composition, préférentiellement de 0,5% à 5%.

La composition utilisable selon l'invention pourra être appliquée par toute voie appropriée, notamment topique externe, et la formulation des compositions sera adaptée par l'homme du métier.

Préférentiellement, les compositions selon l'invention se présentent sous une forme adaptée à l'application par voie topique. Ces compositions doivent donc contenir un milieu physiologiquement acceptable, c'est-à-dire compatible avec la peau et les phanères, sans risque d'inconfort lors de leur application et couvrent toutes les formes cosmétiques adaptées.

Par application topique, on désigne le fait d'appliquer ou d'étaler l'extrait aqueux de *Dunaliella salina* susceptible d'être obtenu par le procédé selon l'invention, et plus particulièrement une composition le contenant, à la surface de la peau, d'une muqueuse ou des phanères.

La « peau » désigne la peau du visage, notamment le contour des yeux et la bouche, le nez, le front, le cou, les mains, mais aussi la peau de l'ensemble du corps.

Les phanères désignent des substances naturellement présentes dans l'organisme humain ou l'organisme animal riches en kératine, et plus particulièrement les cheveux, les poils, les cils, les sourcils et les ongles.

Les compositions topiques pour la mise en oeuvre de l'invention pourront notamment se présenter sous forme d'une solution aqueuse, hydro-alcoolique ou huileuse, d'une émulsion huile-dans-eau, eau-dans-huile, émulsion multiple, micro-émulsion, nano-émulsion ou tout système colloïdal utilisable en cosmétique ; elles peuvent aussi se présenter sous forme de suspensions, ou encore de poudres, adaptées à une application sur la peau, les muqueuses, les lèvres et/ou les cheveux.

Ces compositions peuvent être plus ou moins fluides et avoir également l'aspect d'une crème, d'une lotion, d'un lait, d'un sérum, d'une pommade, d'un gel, d'une pâte ou d'une mousse. Elles peuvent aussi se présenter sous forme solide, comme un stick ou être appliquées sur la peau sous forme d'aérosol.

Dans tous les cas, l'homme de métier veillera à ce que les adjuvants ainsi que leurs proportions soient choisis de telle manière à ne pas nuire aux propriétés avantageuses recherchées de la composition selon l'invention. Ces adjuvants peuvent, par exemple, correspondre à 0,01 à 20% du poids total de la composition. Lorsque la composition selon l'invention est une émulsion, la phase grasse peut représenter de 5 à 80% en poids et de préférence de 5 à 50% en poids par rapport au poids total de la composition. Les émulsionnants et co-émulsionnants utilisés dans la composition sont choisis parmi ceux classiquement utilisés dans le domaine considéré. Par exemple, ils peuvent être utilisés en une proportion allant de 0,3 à 30% en poids par rapport au poids total de la composition.

Un dernier objet de l'invention concerne l'utilisation cosmétique d'une composition selon l'invention pour le soin de la peau, du cuir chevelu et des phanères. L'invention concerne notamment l'utilisation cosmétique de la composition selon l'invention pour améliorer l'aspect de la peau, lutter contre les signes du vieillissement cutané ou pour améliorer l'hydratation de la peau et renforcer la fonction barrière.

L'utilisation et les compositions selon la présente invention sont particulièrement destinées aux soins de la peau et des phanères.

L'invention concerne plus particulièrement l'utilisation cosmétique d'une composition selon l'invention pour améliorer l'hydratation de la peau et renforcer la fonction barrière.

L'invention concerne également plus particulièrement l'utilisation cosmétique d'une composition selon l'invention pour lutter contre les signes du vieillissement cutané et améliorer l'aspect de la peau, en particulier la fermeté et l'élasticité de la peau.

On entend par « améliorer l'aspect de la peau » que le grain de la peau apparait plus fin, la luminosité plus intense et le teint plus homogène.

Par « signes du vieillissement cutané » on entend toutes modifications de l'aspect extérieur de la peau dues au vieillissement comme, par exemple, les rides et ridules, les crevasses, les poches sous les yeux, les cernes, le flétrissement, la perte d'élasticité, de fermeté et/ou de tonus de la peau, mais également toutes modifications internes de la peau qui ne se traduisent pas systématiquement par un aspect extérieur modifié comme, par exemple, l'amincissement de la peau, ou toutes dégradations internes de la peau consécutives à des stress environnementaux tels que la pollution et les rayonnements UV.

On entend par « amélioration de l'hydratation cutanée », toutes améliorations des modifications de l'aspect extérieur de la peau dues à la déshydratation comme, par exemple, la sécheresse, les tiraillements et l'inconfort.

### Exemples

La présente invention va maintenant être illustrée au moyen des exemples suivants :

### Exemple 1 : Préparation d'un extrait de Dunaliella salina fermenté selon l'invention

La préparation de l'extrait de *Dunaliella salina* fermenté va s'effectuer sur deux jours. Le premier jour, le milieu nutritif de culture dans lequel la microalgue va se développer est préparé.

Pour préparer 1 kg de milieu de culture, on place dans un bécher, 0,2% de poudre d'extrait de levure (extrait autolysé de Saccharomyces cerevisiae), soit 2 g de poudre auquel est ajouté environ 800 ml d'eau distillée. La solution est mise sous agitation jusqu'à complète dissolution de la poudre. Une fois la poudre de levure bien solubilisée, 0,2% de glucose est ajouté, soit 2 g de glucose est ajouté à la solution précédemment obtenue. La solution est mise sous agitation constante le temps de la préparation. Une fois le sucre complètement solubilisé, on ajoute 2,6% de sel de la mer Morte, soit 26 g. La solution est maintenue sous agitation jusqu'à complète dissolution du sel. Le pH du milieu nutritif est alors mesuré et doit se situer entre 5,5 et 8 et si besoin est ajusté de façon optimale entre 6 et 7. Le milieu de culture est alors prêt pour accueillir la microalgue *Dunaliella salina.* 2% d'inoculum de *Dunaliello salina* est ajouté au milieu de culture. On entend par inoculum, une culture liquide de *Dunaliella salina* concentrée, dans laquelle les microalgues sont vivantes. De l'eau distillée est ajoutée afin d'obtenir un poids total final de 1 kg. Le pH de la solution finale est alors mesuré et ajusté si besoin entre 6 et 7, pH optimal de culture pour la microalgue *Dunaliella salina.* La solution est maintenue sous agitation et ce pendant minimum 12h à l'obscurité et à température ambiante, en milieu ouvert. Cette culture est dite hétérotrophique, car la fermentation de *Dunaliella salina* a lieu à l'obscurité en présence d'une source carbonée organique. Après la nuit de fermentation des *Dunaliella salina,* le deuxième jour, une étape de broyage permet de libérer le contenu cellulaire des microalgues, afin d'enrichir au maximum le milieu de culture. Par la suite, une étape de filtration avec un filtre contenant du charbon est réalisée afin de désodoriser l'extrait et séparer les débris solides de la phase liquide, et obtenir un extrait limpide. Consécutivement, la purification de l'extrait se fait par l'enchainement de filtrations de porosité décroissante allant de 1 µm à 0,3 µm. Le pH de l'extrait est placé entre 3,5 et 4,5. Enfin, une dernière étape de filtration stérilisante est réalisée d'une porosité de 0,2 µm. L'extrait ainsi obtenu est ce qu'on appelle dans la présente invention la version sans conservateurs, qui a été utilisée pour les tests d'évaluation biologique décrits ci-après dans l'exemple 5.

Optionnellement, des conservateurs peuvent être avantageusement ajoutés à l'extrait ainsi obtenu, par exemple 8 % de sel de la mer morte ou encore du sodium benzoate.

L'extrait de *Dunaliella salina* ainsi obtenu est analysé et présente les caractéristiques suivantes : poids de matière sèche : 22 g/kg ; teneur en protéine totale (fragments protéiques) : 0,5 g/kg ; teneur en sucre totale : 2,3 g/kg ; teneur en acides aminés libres : 0,6 g/kg ; et teneur totale en polyphénols : 40 mg/kg.

La teneur totale en protéines (fragments protéiques) de l'extrait de *Dunaliella salina* a été déterminée par un dosage de protéines de Lowry (Lowry et al., 1951). L'absorbance de l'échantillon est lue sur un spectrophotomètre à 550 nm. La teneur en protéines est déterminée à l'aide d'une courbe standard de BSA.

La teneur en acides aminés de l'extrait a été déterminée à partir d'un protocole publié par Moore (Moore et al., 1948). La teneur en acides aminés libre de l'extrait a été évaluée par la formation d'un complexe coloré, suite à la rupture des fonctions amine et carboxylique par le réactif ninhydrine. L'absorbance du complexe est lue sur un spectrophotomètre à 570 nm. La teneur totale en acides aminés est déterminée par rapport à un pool d'acides aminés comme standard.

La teneur totale en sucres de l'extrait a été déterminée par une adaptation du dosage décrit par Dubois (Dubois et al., "Méthode colorimétrique pour la détermination des sucres et des substances apparentées", Anal. Chem., 1956, 28 (3), 350-356). Cette analyse consiste en la dissolution de la matière première dans l'acide sulfurique concentré puis en réagissant avec du phénol pour former un complexe coloré. L'absorbance du complexe est lue sur un spectrophotomètre à 490 nm. La teneur en sucres est déterminée à l'aide d'une courbe standard de glucose.

La teneur en polyphénols de l'extrait a été déterminée à l'aide du dosage de Folin-Ciocalteu (Singleton et al., Analyse des phénols totaux et d'autres substrats d'oxydation et antioxydants au moyen du réactif folin-ciocalteu, 1999, 299: 152). Les composés de type polyphénols dans l'échantillon réagissent avec le réactif Folin-Ciocalteu, l'oxydation du réactif donne une couleur bleue. L'absorbance de l'échantillon est lue sur un spectrophotomètre à 760 nm. Le contenu a été exprimé en équivalents d'acide gallique à l'aide d'une courbe standard d'acide gallique.

### Exemple 2 : Caractérisation de l'extrait de Dunaliella salina fermenté obtenu selon l'invention

D'une manière générale, on obtient un extrait aqueux de *Dunaliella salina* de couleur vert très pâle, limpide, brillant, titrant de 15 à 25 g/kg d'extrait en poids sec, 0,1 à 2 g/kg de fragments protéiques, 0,3 à 3 g/kg de sucres, 0,5 à 3 g/kg d'acides aminés, et 20 à 150 mg/kg de composés phénoliques.

Dans l'extraction de l'exemple 1, on a obtenu un extrait aqueux titrant 22 g/kg d'extrait de poids sec. Cet extrait est appelé extrait « fermenté non-conservé » et a été utilisé pour toutes les analyses biologiques.

L'analyse physico-chimique montre que cet extrait présente une concentration de 0,5 g/kg de fragments protéiques, 2,3 g/kg de sucres, 640 mg/kg d'acides aminés, et 40 mg/kg de composés phénoliques.

Optionnellement, l'extrait ainsi obtenu peut être avantageusement conservé par ajout de sel de la mer Morte à une concentration de 8% ou encore par un conservateur tel que du sodium benzoate. L'extrait est alors dit « extrait conservé ».

### Exemple 3 : Réalisation d'un extrait de Dunaliella salina « non fermenté »

Pour obtenir un extrait de *Dunaliella salina* dit « non fermenté », la réalisation de l'extrait se fait par le procédé tel que décrit dans l'exemple 1, la seule différence étant que la solution contenant le milieu de culture et la microalgue n'est pas mise sous agitation pendant 12h à l'obscurité, période durant laquelle la microalgue fermente. Dès que la microalgue est ajoutée au milieu, la solution contenant le milieu nutritif et la microalgue est immédiatement broyée et filtrée tel que décrit dans l'exemple 1. Cela permet d'obtenir un extrait dans lequel la microalgue n'a pas effectué de fermentation hétérotrophique et ainsi d'effectuer des analyses comparatives tant d'un point de vue physico-chimique qu'au niveau de l'évaluation biologique.

### Exemple 4 : Mise en évidence de la fermentation de Dunaliella salina dans les conditions de culture de l'exemple 1

Afin de mettre en évidence que la microalgue *Dunaliella salina* est capable de se développer dans les conditions de culture décrites selon l'invention (exemple 1), des modifications de certains paramètres de culture et des analyses de certains marqueurs biologiques ont été effectuées.

L'un des premiers paramètres de culture à mesurer quand on suit la croissance d'un microorganisme en culture est sa biomasse au cours du temps. La densité cellulaire est mesurée par densité optique (DO) qui est un paramètre fiable pour la mesure de la croissance. Pour cela, la DO a été mesurée par spectrophotométrie à une longueur d'onde de 700 nm communément utilisée pour définir l'opacité d'une culture d'un microorganisme.

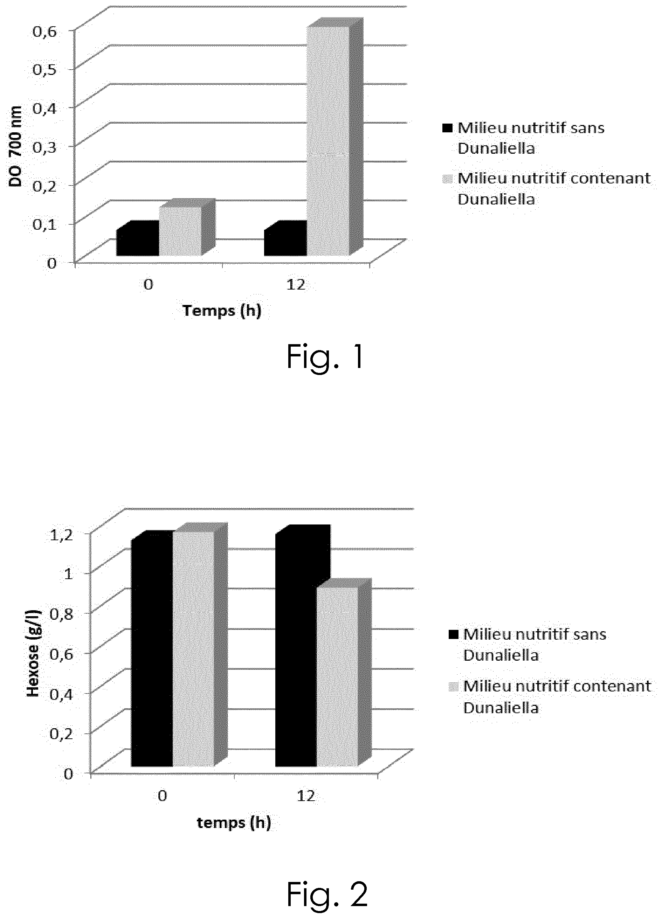

La figure 1 montre la DO mesurée à l'obscurité et à température ambiante à t=0 et t=12h du milieu de culture seul (ou milieu nutritif), c'est-à-dire sans addition de la microalgue *Dunaliella salina,* et du milieu de culture additionné de la microalgue *Dunaliella salina.* On observe une augmentation significative de la DO après 12h en présence de *Dunaliella salina.* Cette augmentation est due à la croissance des *Dunaliella salina* qui ont fermenté et se sont divisées pendant ce laps de temps de 12h.

Il est également possible de mettre en évidence la croissance de microorganisme en culture par leur consommation en substances nécessaires à leur croissance. C'est le cas du sucre, qui est un élément nutritif indispensable à la division cellulaire des microorganismes. En effet, la consommation de la source carbonée du milieu de culture, par exemple le glucose (hexose), est un paramètre de suivi de culture reconnu pour le suivi de la croissance des microorganismes. Une analyse comparative de la concentration en hexose dans le milieu de culture contenant ou non la microalgue *Dunaliella salina* à température ambiante à t=0 et t=12h en absence de lumière et à température ambiante a été effectuée. La teneur totale en hexose de l'extrait a été déterminée d'après la méthode de Hanssen et Moller (1975) dans laquelle la quantité de sucres en C6 est déterminée par un dosage colorimétrique. Une solution d'anthrone solubilisée dans de l'acide sulfurique va réagir spécifiquement avec les sucres en virant du jaune au vert. L'absorbance du complexe est lue sur le spectrophotomètre à 625 nm. La teneur en sucre est déterminée à l'aide d'une courbe standard de glucose.

Les résultats présentés sur la figure 2 montrent une diminution de la concentration en glucose dans le milieu de culture à t=12h, et ce uniquement dans le milieu de culture contenant *Dunaliella salina,* signifiant que les microalgues ont consommé du glucose et l'ont métabolisé.

Les acides aminés sont une source azotée importante pour les microalgues qui poussent dans les milieux naturels. Il a été démontré que les acides aminés forment une proportion significative de l'azote dissous dans les eaux marines et pourraient fournir une source supplémentaire d'azote pour la croissance des microalgues marines. Certaines études suggèrent que les taux maximaux de capture d'acides aminés se produisent dans des conditions d'obscurité dans les eaux appauvries en azote inorganique dissous.

La capacité de *Dunaliella salina* à pousser en condition hétérotrophique avec comme source nutritive carbonée du glucose et comme source azotée un extrait de levure très riche en molécules azotées tels que les acides aminés a été évaluée. La culture dans ce cas se fait dans l'obscurité, propice à la métabolisation des acides aminés présents dans le milieu de culture.

Afin de déterminer précisément les acides aminés contenus dans le milieu de culture à température ambiante à t=0 et t=12h en l'absence de lumière, les échantillons ont été analysés par HPLC-DAD. Les milieux de culture contenant les microalgues *Dunaliella salina* à t=0 et t=12h ont été centrifugés afin d'ôter les microalgues, pour ne doser que les acides aminés présents dans le milieu de culture. Les échantillons ont été séparés par une Colonne Uptisphere Strategy C18-2 5µm (250x4.6mm) US5C182-250/046 (référence Interchim: UE2.6AQ-100/046) sur une Agilent 1260 HPLC system (Agilent Technologies, CA, USA). Le débit étant de 1 ml/min. La phase mobile consiste en de l'acide phosphorique (H3PO4) à 0,1% : solution (A) et de l'acétonitrile : solution (B). Le tableau 1 ci-dessous représente le programme des gradients pour l'élution.

**Tableau 1 :**

| Temps (min) | % (H3PO4 0,1%) | % (ACN) |
|---|---|---|
| 0 | 87 | 13 |
| 20 | 54 | 46 |
| 22 | 54 | 46 |
| 27 | 60 | 40 |
| 30 | 60 | 40 |
| 35 | 5 | 95 |
| 37 | 87 | 13 |

La température de la colonne a été maintenue à 25°C. Le volume d'injection a été de 5 µL et la longueur d'onde de détection a été fixée à 254 nm en utilisant un détecteur UV. Les standards d'acides aminés ont été achetés auprès de Sigma-Aldrich. Les standards et l'échantillon avant injection sont dérivés avec du phénylisothiocyanate (PITC). L'identification des acides aminés a été réalisée en comparant les temps de rétention et les pics spectraux UV de l'échantillon par rapport aux acides aminés standards.

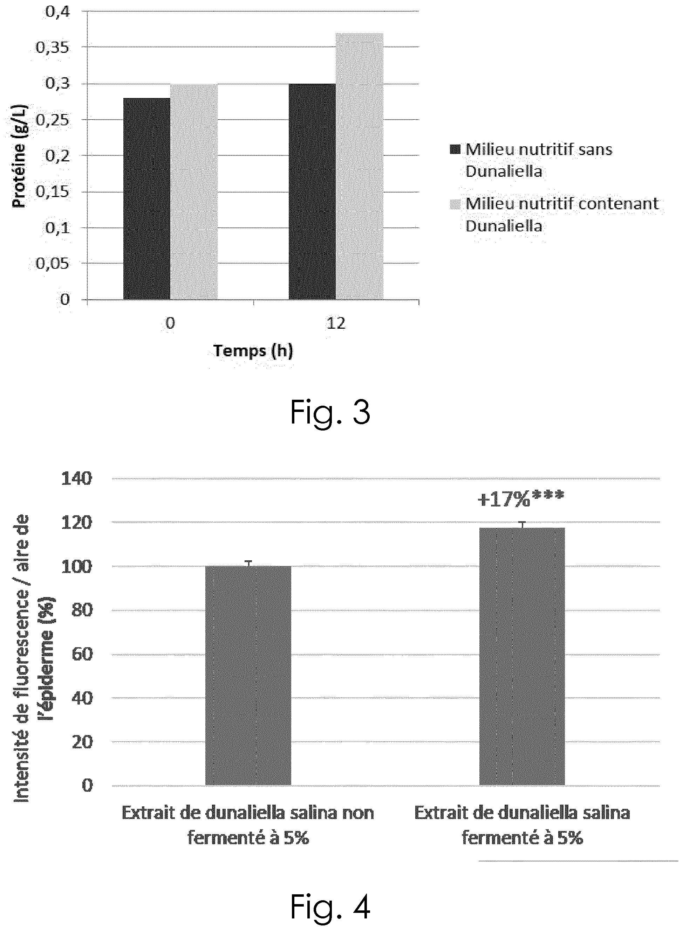

La concentration de certains acides aminés a diminué de façon significative dans la condition d'un milieu de culture contenant *Dunaliella salina* après t=12h de fermentation par rapport au temps t=0, en particulier pour la serine, asparagine, acide aspartique, alanine, arginine, acide glutamique. Ces différents acides aminés ont été consommés par les microalgues au cours de la fermentation, et ont servi en partie à la synthèse des protéines de la microalgue en croissance. Ceci est mis en évidence par la figure 3 où le taux de protéines d'un extrait « microalgue plus milieu nutritif» est supérieur après 12h de fermentation.

### Exemple 5 : Evaluation de l'effet de l'extrait aqueux de Dunaliella salina fermenté, préparé selon l'exemple 1, sur la fonction barrière et les mouvements de l'eau sur de la peau ex vivo

Le but de cette étude est de montrer l'effet de l'extrait aqueux de *Dunaliella* salina fermenté, préparé selon l'exemple 1, sans ajout de conservateurs, comparé à l'extrait aqueux de *Dunaliella salina* non fermenté, préparé selon l'exemple 3, sur la fonction barrière et les mouvements de l'eau par l'évaluation de la claudine-1.

La peau constitue l'interface entre l'organisme et l'environnement extérieur. Elle a pour but de protéger l'organisme des agressions extérieures mais également de lutter contre la déshydratation en limitant la diffusion de l'eau. Le maintien de l'homéostasie hydrique est assuré par différents éléments dont les jonctions serrées de la couche granuleuse. Ces jonctions, constituées en partie de la protéine transmembranaire claudine-l, empêchent la diffusion passive des solutés et de l'eau par l'espace intercellulaire. Enfin, on a observé que l'expression de la claudine-1 diminue avec le vieillissement de la peau et la déficience en claudine-1 conduit à l'apparition de rides (Furuse et al.,"Claudin-based tight junctions are crucial for the mammalian epidermal barrier: a lesson from claudin-1-deficient mice", J Cell Biol. 156(6):1099-111, 2002; Jin S.P. et al., "Changes in tight junction protein expression in intrinsic aging and photoaging in human skin in vivo", J DermatolSci. 97-113, 2016).

### Protocole :

Des biopsies de peau humaine de 6 mm de diamètre ont été maintenues ex *vivo* dans un milieu de culture spécifique (DMEM à 1 g/L, HAMF12, sérum de veau fœtal et antibiotiques). Les biopsies ont été traitées 2 fois par jour pendant 48 heures avec soit une solution d'extrait de *Dunaliella salina* fermenté, préparé selon l'exemple 1, soit une solution d'extrait de *Dunaliella salina* non fermenté, préparé selon l'exemple 3, diluées toutes les 2 dans du PBS pour avoir une concentration finale de 5% vol/vol.

Pour l'immunomarquage de la claudine-1, les tissus sont fixés et inclus dans de la paraffine. Les biopsies de peau ainsi incluses sont coupées et les sections sont déparaffinées puis réhydratées. Ensuite, un protocole de démasquage est réalisé avant l'application d'un anticorps spécifique anti-claudine-1 (Abcam, ab15098, polyclonal de lapin), puis d'un anticorps secondaire adapté, couplé à un colorant fluorescent. Après avoir été monté dans un milieu particulier, les lames sont observées au microscope à épifluorescence (Zeiss Axiovert 200M microscope).

### Résultats :

Comme illustré par la figure 4, le traitement avec la solution d'extrait de *Dunaliella salina* fermenté diluée à 5% dans du PBS a montré une augmentation significative (^{∗∗∗}) de 17% de l'expression de la claudine-1 comparé à la solution d'extrait de *Dunaliella salina* non fermenté diluée à 5% dans du PBS, dans des biopsies de peau ex *vivo.*

### Conclusion :

Après application d'une solution d'extrait de *Dunaliella salina* fermenté diluée à 5%, nous observons une augmentation d'expression de la claudine-1. La claudine-1 est associée à la fonction barrière et aux mouvements de l'eau dans la peau.

### Exemple 6 : Formulations

Dans les formules suivantes, l'extrait de *Dunaliella salina* fermenté utilisé a été obtenu selon l'exemple 1 et additionné de 8% de sel de la mer Morte comme conservateur. Il est appelé : Jus minéral de fermentation (FERMENT MINERAL BROTH).

### Exemple 6.1 : Crème de jour hydratante

Phase A

| Eau | qsp |
|---|---|
| Glycérine | 3 |
| Aloe Vera | 0,1 |
| Hyaluronate de sodium | 0,2 |
| Ascorbyl glucoside | 3 |

Phase B

| | |
|---|---|
| OLIVEM 1000^{®} = Cétéaryle Olivate, Sorbitan Olivate | 5 |
| OLIWAX^{®} LC = Cétyle Palmitate, Sorbitan Palmitate, Sorbitan Olivate | 1 |
| Huile d'argan | 1 |
| Phytosqualane | 4 |
| Vitamine E = Tocophéryle Acétate | |

Phase C

| | |
|---|---|
| Conservateur | 1 |

Phase D

| | |
|---|---|
| Fragrance | 0,6 |
| Jus minéral de fermentation (FERMENT MINERAL BROTH) | 1 |

### Exemple 6.2 : Sérum pour le visage

Phase A

| Eau | qsp |
|---|---|
| Glycérine | 3 |
| Aloe Vera | 0,1 |
| Hyaluronate de sodium | 0,1 |
| Ascorbyl glucoside | 5 |

Phase B

| | |
|---|---|
| SIMULGEL^{®} INS 100 = Hydroxyéthyle Acrylate/Sodium Acryloyldiméthyle Taurate Copolymère (et) Isohexadecane (et) Polysorbate | 60 |
| Huile de pépins de raisin | 1 |
| Phytosqualane | 4 |
| Vitamine E = Tocophéryl Acétate | |

Phase C

| | |
|---|---|
| Jus minéral de fermentation (FERMENT MINERAL BROTH) | 2 |
| Conservateur | 1 |

Phase D

| | |
|---|---|
| Fragrance | 0,6 |

### Exemple 6.3 : Essence pour le visage

### Phase A

| Eau | qsp |
|---|---|
| Jus minéral de fermentation (FERMENT MINERAL BROTH) | 30 |
| Glycérine | 3 |
| FUCOGEL^{®} = Biosaccharide Gum-1 | 1 |
| Fragrance | 0,3 |
| Extrait d'algue rouge | 0,01 |

### Exemple 6.4 : Masque de nuit

Phase A

| Eau | qsp |
|---|---|
| Extrait de cactus | |
| Lactate de sodium | |
| Aloe vera | |
| Allantoine | |

Phase B

| | |
|---|---|
| TEGOCARE^{®} 450 = Polyglycéryl-3 Méthylglucose Distéarate | 1 |
| DERMOWAX^{®} SB = Stéaryl Béhénate | 0,2 |
| INUTEC^{®} SL1 = Glycérine, Inuline Lauryl Carbamate | 0,2 |
| Isononyle isononanoate | 0,5 |

Phase C

| | |
|---|---|
| Conservateur | 0,5 |
| TWEEN^{®} 20 = Polysorbate 20 | 0,1 |
| Huile essentielle de lavande | 0,5 |
| Phase D | |
| Jus minéral de fermentation (FERMENT MINERAL BROTH) | 5 |

### Exemple 6.5 : Gel douche de la mer Morte

Phase A

| Eau | qsp |
|---|---|
| Glycérine | 10 |
| POLYQUATERNIUM-10 | 0,3 |
| Acide citrique | 0,5 |
| JAGUAR^{®} C17 = Guar Hydroxypropyltrimonium Chloride | 0,3 |

Phase B

| | |
|---|---|
| MIRACARE^{®} SLB 365W = Eau (et) Sulfate de tridéceth de sodium (et) Cocamide MEA (et) Sodium Lauroamphoacétate (et) Chlorure de sodium | 28 |

Phase C

| | |
|---|---|
| Huile de jojoba | 3 |
| Fragrance | 1 |

Phase D

| | |
|---|---|
| Conservateur EUXYL^{®} 9010 = Phénoxyéthanol (et) Ethylhexylglycérine | 1 |

Phase E

| | |
|---|---|
| Jus minéral de fermentation (FERMENT MINERAL BROTH) | 1 |

### Exemple 6.6 : Shampooing de la mer Morte

Phase A

| Eau | qsp |
|---|---|
| POLYQUATERNIUM-10 | 0,2 |
| Panthénol | 0,15 |
| ELFACOS^{®} GT 282S = Cétéareth-60 Myristyl Glycol | 0,7 |
| Acide citrique | 0,25 |

Phase B

| | |
|---|---|
| Eau | 1 |
| Boue de la mer Morte | 0,5 |
| Jus minéral de fermentation (FERMENT MINERAL BROTH) | 1 |

Phase C

| | |
|---|---|
| Sulfate de laureth de sodium | 8,5 |
| MIRANOL^{®} C2M = Disodium Cocoamphodiacétate | 12 |
| REWODERM^{®} S1333 = Disodium Ricinoléamido MEA-Sulfosuccinate | 2,1 |

Phase D

| | |
|---|---|
| EUXYL^{®} 9010 = Phénoxyéthanol (and) Ethylhexylglycérine | 1,1 |
| Tocophéryl acétate | 0,05 |
| Huile d'argan | 0,1 |
| Fragrance | 0,8 |

### Exemple 6.7 : Revitalisant cheveux de la mer Morte

Phase A

| Eau | qsp |
|---|---|
| Glycérine | 1 |
| Niacinamide | 0,2 |
| Chlorure de behentrimonium | 2 |
| POLYQUATERNIUM-10 | 0,3 |
| DOW CORNING^{®} 939 émulsion = Amodiméthicone (et) chlorure de cétrimonium (et) TRIDECETH-12^{®} | 1 |

Phase B

| | |
|---|---|
| Isopropyl myristate | 2 |
| Huile d'argan | 1 |
| Alcool cétéaryl | 5 |
| DOW CORNING^{®} 200 fluide = Diméthicone | 1,5 |

Phase C

| | |
|---|---|
| Eau | 1 |
| Boue de la mer Morte | 1 |
| Jus minéral de fermentation (FERMENT MINERAL BROTH) | 1 |

Phase D

| | |
|---|---|
| EUXYL^{®} 9010 = Phénoxyéthanol (et) Ethylhexylglycérine | 1,1 |
| Tocophéryl acétate | 0,05 |
| Fragrance | 1 |

Phase E

| | |
|---|---|
| Extrait de thé vert | 0,5 |
| Extrait de camomille | 0,5 |
| Extrait de grenade | 0,5 |
| SILK TEIN^{®} NPNF = soie hydrolysée | 0,5 |

### Exemple 6.8 : Masque capillaire de la mer Morte

Phase A

| Eau | qsp |
|---|---|
| Glycérine | 3 |
| Acide lactique | 1 |
| Panthénol | 1 |
| Chlorure de Behentrimonium | 1,5 |
| DOW CORNING^{®} 939 émulsion = Amodiméthicone (et) chlorure de cétrimonium (et) TRIDECETH-12^{®} | 4 |
| POLYQUATERNIUM-10 | 2 |

Phase B

| | |
|---|---|
| Cyclopentasiloxane SF 1202 | 2 |
| ABIL QUAT^{®} 3474 Quaternium-80 | 2 |
| MYRITOL^{®} 318 = Caprylique/Caprique Triglycéride | 0,5 |
| Huile d'argan | 0,1 |
| Alcool cétéaryl | 9,5 |
| Glycéryl stéarate | 1 |

Phase C

| | |
|---|---|
| EUXYL^{®} 9010 = Phénoxyéthanol (et) Ethylhexylglycérine | 1,1 |

Phase D

| | |
|---|---|
| Boue de la mer Morte | 1 |
| Jus minéral de fermentation (FERMENT MINERAL BROTH) | 5 |

Phase E

| | |
|---|---|
| Fragrance | 1 |
| Tocophéryl acétate | 0,5 |

Phase F

| | |
|---|---|
| Protéine G de soie | 0,1 |
| FISION KERAVEG^{®} 18 = Eau (et) Acides aminés de blé (et) Acides aminés de soja (et) Arginine HCl (et) Sérine (et) Thréonine | 0,7 |
| Agent de coloration | 0,1 |

## Revendications

1. Procédé d'obtention d'un extrait aqueux d'une microalgue *Dunaliella salina,* dans lequel ladite microalgue est cultivée en absence de lumière dans un milieu de culture comprenant un extrait de levure, du sucre et du sel suivant les étapes selon lesquelles :
a) on solubilise un extrait de levure dans de l'eau ;
b) on ajoute du glucose dans le mélange obtenu en a) ;
c) on ajoute du sel de la mer Morte ;
d) après totale solubilisation du sel dans le mélange obtenu en b), le milieu de culture ainsi obtenu présente un pH compris entre 5 et 8 dans lequel on ajoute la microalgue *Dunaliella salina ;*
e) on maintient le mélange obtenu en d) sous agitation modérée, à l'obscurité et à température ambiante, pendant une période d'au moins 12h, pour permettre la fermentation de la microalgue *Dunaliella salina* ;
f) le mélange de *Dunaliella salina* fermenté obtenu en e) est broyé puis filtré afin de séparer les matières solubles et insolubles ;
g) on récupère un extrait brut aqueux soluble dans lequel on rajoute du sel de la mer Morte ou un conservateur tel que du sodium benzoate ;
h) on réalise une filtration stérilisante avec un seuil de porosité inférieur ou égal à 0,2 µm ; et
i) on obtient un extrait aqueux de *Dunaliella salina* fermenté dans lequel le pH est compris entre 3,5 et 4,5.

2. Procédé selon la revendication 1, dans lequel dans l'étape a) on solubilise un extrait de levure sous forme de poudre dans de l'eau distillée suivant un ratio extrait de levure / eau de 0,1 à 2% en poids/poids.

3. Procédé selon la revendication 2, dans lequel dans l'étape a) on solubilise un extrait de levure sous forme de poudre dans de l'eau distillée suivant un ratio extrait de levure / eau de 0,2% en poids/poids.

4. Procédé selon l'une des revendications 1 à 3, dans lequel dans l'étape b) le glucose est ajouté à une concentration comprise entre 0,1 et 4% en poids du poids total du mélange.

5. Procédé selon la revendication 4, dans lequel dans l'étape b) le glucose est ajouté à une concentration de 2% en poids du poids total du mélange.

6. Procédé selon l'une des revendications précédentes, dans lequel dans les étapes d) et i) le pH est contrôlé et éventuellement réajusté par l'ajout d'une solution d'acide chlorhydrique (HCl) ou de soude (NaOH).

7. Procédé selon l'une des revendications précédentes, dans lequel dans l'étape i) le pH est compris entre 3,5 et 4,0.

8. Procédé selon la revendication 7, dans lequel dans l'étape i) le pH est de 4,0.

9. Extrait aqueux de *Dunaliella salina* susceptible d'être obtenu par le procédé selon l'une des revendications 1 à 8, **caractérisé en ce qu'**il comprend en poids du poids total de l'extrait, de 15 à 25 g/kg de poids sec, 0,1 à 2 g/kg de fragments protéiques, 0,3 à 3 g/kg de sucres, 0,5 à 3 g/kg d'acides aminés et 20 à 150 mg/kg de composés phénoliques.

10. Composition comprenant, en tant qu'agent actif anti-âge, une quantité efficace d'un extrait aqueux de *Dunaliella salina* selon la revendication 9, et un milieu physiologiquement acceptable.

11. Composition selon la revendication 10, **caractérisée en ce qu'**elle comprend l'extrait aqueux de *Dunaliella salina* à une concentration de 0,1 à 10% en poids par rapport au poids total de la composition.

12. Composition selon la revendication 11, **caractérisée en ce qu'**elle comprend l'extrait aqueux de *Dunaliella salina* à une concentration de 0,5% à 5% en poids par rapport au poids total de la composition.

13. Utilisation cosmétique d'une composition selon l'une des revendications 10 à 12 pour le soin de la peau, du cuir chevelu et des phanères.

14. Utilisation cosmétique selon la revendication 13 pour améliorer l'aspect de la peau, lutter contre les signes du vieillissement cutané ou pour améliorer l'hydratation de la peau et renforcer la fonction barrière.

## Patentansprüche

1. Verfahren zur Gewinnung eines wässrigen Extrakts einer Mikroalge *Dunaliella salina,* bei dem die Mikroalge in Abwesenheit von Licht in einem Kulturmedium gezüchtet wird, das einen Hefeextrakt, Zucker und Salz umfasst, nach den Schritten, gemäß denen:
a) ein Hefeextrakt in Wasser gelöst wird;
b) Glucose zu der in a) erhaltenen Mischung hinzugefügt wird;
c) Salz aus dem Toten Meer hinzugefügt wird;
d) nach vollständiger Solubilisierung des Salzes in der in b) erhaltenen Mischung das so erhaltene Kulturmedium einen pH-Wert zwischen 5 und 8 aufweist, dem die Mikroalge *Dunaliella salina* zugesetzt wird;
e) die unter d) erhaltene Mischung im Dunkeln und bei Raumtemperatur über einen Zeitraum von mindestens 12 Stunden mäßig gerührt wird, um die Fermentation der Mikroalge *Dunaliella salina* zu ermöglichen;
f) die in e) erhaltene Mischung aus fermentiertem *Dunaliella salina* wird gemahlen und dann filtriert, um die löslichen und unlöslichen Stoffe zu trennen;
g) ein löslicher wässriger Rohextrakt gewonnen wird, dem Salz aus dem Toten Meer oder ein Konservierungsmittel wie Natriumbenzoat zugesetzt wird;
h) eine Sterilisationsfiltration mit einer Porositätsschwelle kleiner oder gleich 0,2 µm wird durchgeführt und
i) ein wässriger Extrakt aus fermentierter *Dunaliella salina* wird erhalten, bei dem der pH-Wert zwischen 3,5 und 4,5 liegt.

2. Verfahren nach Anspruch 1, wobei in Schritt a) ein Hefeextrakt in Pulverform in destilliertem Wasser gemäß einem Hefeextrakt/Wasser-Verhältnis von 0,1 bis 2 Gew.-% gelöst wird.

3. Verfahren nach Anspruch 2, wobei in Schritt a) ein Hefeextrakt in Pulverform in destilliertem Wasser gemäß einem Hefeextrakt/Wasser-Verhältnis von 0,2 Gew.-% gelöst wird.

4. Verfahren nach einem der Ansprüche 1 bis 3, wobei in Schritt b) Glucose in einer Konzentration zwischen 0,1 und 4 Gew.-% des Gesamtgewichts der Mischung zugegeben wird.

5. Verfahren nach Anspruch 4, wobei in Schritt b) Glucose in einer Konzentration von 2 Gew.-% des Gesamtgewichts der Mischung zugegeben wird.

6. Verfahren nach einem der vorstehenden Ansprüche, wobei in den Schritten d) und i) der pH-Wert überprüft und gegebenenfalls durch Zugabe einer Salzsäure-(HCl) oder Natriumhydroxidlösung (NaOH) neu eingestellt wird.

7. Verfahren nach einem der vorstehenden Ansprüche, wobei in Schritt i) der pH-Wert zwischen 3,5 und 4,0 liegt.

8. Verfahren nach Anspruch 7, wobei in Schritt i) der pH-Wert 4,0 beträgt.

9. Wässriger Extrakt von *Dunaliella salina,* der möglicherweise durch das Verfahren nach einem der Ansprüche 1 bis 8 erhalten werden kann, **dadurch gekennzeichnet, dass** er, bezogen auf das Gewicht des Gesamtgewichts des Extrakts 15 bis 25 g/kg Trockengewicht, 0,1 bis 2 g/ kg Proteinfragmente, 0,3 bis 3 g/kg Zucker, 0,5 bis 3 g/kg Aminosäuren und 20 bis 150 mg/kg phenolische Verbindungen umfasst.

10. Zusammensetzung, umfassend als Anti-Aging-Wirkstoff eine wirksame Menge aus einem wässrigen Extrakt von *Dunaliella salina* nach Anspruch 9 und einem physiologisch verträglichen Medium.

11. Zusammensetzung nach Anspruch 10, **dadurch gekennzeichnet, dass** sie den wässrigen Extrakt von *Dunaliella salina* in einer Konzentration von 0,1 bis 10 Gew.-%, bezogen auf das Gesamtgewicht der Zusammensetzung umfasst.

12. Zusammensetzung nach Anspruch 11, **dadurch gekennzeichnet, dass** sie den wässrigen Extrakt von *Dunaliella salina* in einer Konzentration von 0,5 bis 5 Gew.-%, bezogen auf das Gesamtgewicht der Zusammensetzung umfasst.

13. Kosmetische Verwendung einer Zusammensetzung nach einem der Ansprüche 10 bis 12 zur Pflege der Haut, der Kopfhaut und der Hautanhangsgebilde.

14. Kosmetische Verwendung nach Anspruch 13 zur Verbesserung des Hautbildes, zur Bekämpfung von Hautalterungserscheinungen oder zur Verbesserung der Feuchtigkeitsversorgung der Haut und Stärkung der Hautbarrierefunktion.

## Claims

1. A method for obtaining an aqueous extract a *Dunaliella salina* microalgae in which said microalgae is cultivated in the absence of light in a culture medium comprising a yeast extract, sugar and salt according to the following steps:
a) solubilising a yeast extract in water;
b) adding glucose to the mixture obtained in a);
c) adding Dead Sea salt;
d) after total solubilisation of the salt in the mixture obtained in b), the culture medium thus obtained has a pH of between 5 and 8, and the microalgae *Dunaliella salina* is added thereto;
e) moderately stirring the mixture obtained in d), in darkness and at room temperature, for a period of at least 12 hours, in order to allow the fermentation of the microalgae *Dunaliella salina;*
f) grinding and then filtering the fermented *Dunaliella salina* mixture obtained in e) in order to separate the soluble and insoluble materials;
g) recovering a soluble aqueous raw extract to which Dead Sea salt or a preservative such as sodium benzoate is added;
h) carrying out sterilising filtration with a porosity threshold of less than or equal to 0.2 µm; and
i) obtaining a fermented aqueous extract of *Dunaliella salina* in which the pH is between 3.5 and 4.5.

2. The method according to claim 1, wherein in step a) a yeast extract in powder form is solubilised in distilled water in a ratio of yeast extract/water of 0.1 to 2% w/w.

3. The method according to claim 2, wherein in step a) a yeast extract in powder form is solubilised in distilled water in a ratio of yeast extract/water of 0.2% w/w.

4. The method according to claims 1 to 3, wherein in step b) glucose is added in a concentration of between 0.1 and 4% by weight of the total weight of the mixture.

5. The method according to claim 4, wherein in step b) glucose is added in a concentration of 2% by weight of the total weight of the mixture.

6. The method according to any one of the preceding claims, wherein in steps d) and i) the pH is controlled and optionally readjusted by adding a solution of hydrochloric acid (HCI) or sodium hydroxide (NaOH).

7. The method according to any one of the preceding claims, wherein in step i) the pH is between 3.5 and 4.0.

8. The method according to claim 7, wherein in step i) the pH is 4.0.

9. An aqueous extract of *Dunaliella salina* likely to be obtained by the method according to any one of claims 1 to 8, wherein it comprises, by weight of the total weight of the extract, from 15 to 25 g/kg dry weight, 0.1 to 2 g/kg protein fragments, 0.3 to 3 g/kg sugars, 0.5 to 3 g/kg amino acids and 20 to 150 mg/kg phenolic compounds.

10. A composition comprising, as an active anti-aging agent, an effective amount of an aqueous extract of *Dunaliella salina* according to claim 9, and a physiologically acceptable medium.

11. The composition according to claim 10, wherein it comprises the aqueous extract of *Dunaliella salina* at a concentration of 0.1 to 10% by weight of to the total weight of the composition.

12. The composition according to claim 11, wherein it comprises the aqueous extract of *Dunaliella salina* at a concentration of 0.5 to 5% by weight of to the total weight of the composition.

13. A cosmetic use of a composition according to any one of claims 10 to 12 for skincare, scalp care and skin appendage care.

14. The cosmetic use according to claim 13 for improving the appearance of the skin, combating the signs of skin aging or for improving the hydration of the skin and strengthening the barrier function.
